# EUROPÄISCHE PATENTSCHRIFT

(11) **EP 1 696 905 B1**
(45) Veröffentlichungstag und Bekanntmachung des Hinweises auf die Patenterteilung: **19.03.2008**
(21) Anmeldenummer: 04804250.1
(22) Anmeldetag: 23.12.2004
(51) Int. Cl.: A61K 31/381, A61P 25/16

(54) **SUBSTITUIERTE 2-AMINOTETRALINE ZUR VORBEUGENDEN BEHANDLUNG VON MORBUS PARKINSON**
SUBSTITUTED 2-AMINOTETRALINES FOR THE PREVENTIVE TREATMENT OF PARKINSON'S DISEASE
2-AMINOTETRALINES SUBSTITUEES POUR LE TRAITEMENT PROPHYLACTIQUE DE LA MALADIE DE PARKINSON

(30) Priorität: 24.12.2003 DE 10361258
(43) Veröffentlichungstag der Anmeldung: 06.09.2006
(73) Patentinhaber: SCHWARZ PHARMA AG, 40789 Monheim/Rhld. (DE)
(72) Erfinder: SCHELLER, Dieter, 41470 Neuss (DE); DRESSEN, Frank, 52391 Vettweiss (DE)
(74) Vertreter: HOFFMANN EITLE
(86) Internationale Anmeldenummer: PCT/EP2004/014656
(87) Internationale Veröffentlichungsnummer: WO 2005/063238

(56) Entgegenhaltungen:
- WO-A-00/38669
- WO-A-02/089777
- RIEDERER ET AL.: "NEUROPROTECTION THE LUGANO CONSENSUS" J. NEUROL., Bd. 247, Nr. 4, 2000, Seiten IV36-IVF37, XP002327044

## Beschreibung

Die Parkinson'sche Krankheit ist die Folge einer chronisch-fortschreitenden Degeneration von Neuronen, deren Ursache noch nicht völlig aufgeklärt ist. Sie wird klinisch manifest in Form der Kardinalsymptome Ruhetremor, Rigor, Bradykinesie und posturale Instabilität.

Als Medikamente zur Linderung der motorischen Symptome werden in erster Linie Levodopa, Dopaminagonisten, wie z.B. Rotigotin, Pramipexol, Bromocriptin, Ropinirol, Cabergoline, Pergolid, Apomorphin und Lisurid, Anticholinergika, NMDA-Antagonisten, β-Blocker sowie der MAO-B-Inhibitor Selegelin und der COMT-Inhibitor Entacapone eingesetzt. Diese meisten dieser Wirkstoffe greifen in die dopaminerge und/oder cholinerge Signalkaskade ein und beeinflussen auf diese Weise symptomatisch die Parkinson-typischen Bewegungsstörungen.

Die bisherige Therapie der Parkinson'schen Krankheit setzt beim Eintreten der Kardinalsymptome ein. Eine Parkinson'sche Krankheit gilt klinisch im allgemeinen als nachgewiesen, wenn mindestens zwei der vier Kardinalsymptome (Bradykinesie, Ruhetremor, Rigor und posturale Instabilität) zu finden sind und L-Dopa anspricht (Hughes, J Neurol Neurosurg Psychiatry 55,1992,181). Unglücklicherweise treten die motorischen Störungen bei Parkinson-Patienten aber erst auf, wenn ca. 70-80 % der dopaminergen Neurone in der Substanzia Nigra (SN) irreversibel geschädigt sind (Becker et al, J Neurol 249, 2002, Suppl 3:III, 40; Hornykiewicz, Encyclopedia of Life Science 2001, 1). Die Chance einer nachhaltigen Therapie zu diesem Zeitpunkt sind nur noch gering. Daher ist es wünschenswert, die Therapie so früh wie möglich einsetzen zu lassen.

Aktuelle klinische Beobachtungen sowie anatomische und genetische Untersuchungen zeigen nun, dass sowohl die Diagnose von Parkinsonpatienten in frühen Stadien als auch die Identifizierung von Risikopatienten möglich ist.

Als diagnostische Marker können dabei beispielsweise herangezogen werden:
- Biochemische Marker, wie Neuromelanin (Gerlach, Neurotox Res 5,2003, 35; WO 02/31499), S-100 beta (Muramatsu, Glia 42, 2003, 307), das alpha-Synuclein (WO 03/069332; WO 00/02053) oder das parkin-Protein (Sharma, Neurol Clin N Am 20, 2002, 759) sowie Semaphorin (Wo 03/007803)
- Genetische Marker, z.B. die Park-Gene 1-8 (Guttman, CMAJ 4, 2003, 168); CYP2D6-B (WO 03/012137), Chromosom 2q 36-37 (Pankratz, Am J Hum Gen 72; 2003,e-pub), a-Synuclein (Polymeropoulos, Science. 276, 1997, 2045) oder Mutationen in CYP2D6-B und GSTM1-Deletion (WO 03/012137).
- Bildgebende Verfahren, wie die Ultraschalluntersuchung der SN-Größe, gegebenenfalls in Kombination mit anderen Verfahren (Becker et al, J Neurol 249, 2002, Suppl 3:III, 40) oder MRI (Hutchinson M, Raff U., J Neurol Neurosurg Psychiatry. 1999 Dec;67(6):815-8).
- Bildgebende Verfahren wie PET oder SPECT (Prunier C, Bezard E, et al., Neuroimage. 2003 Jul;19(3):810-6).
- Sensorische Störungen oder Verhaltensauffälligkeiten, wie Schlaf- und Riechstörungen, insbesondere Schlafstörungen vom Typ der ,REM Behavior Disorder', (Henderson, J Neurol Neurosurg Psychiatry 74, 2003, 956) oder kognitive Abnormalitäten (Rammsayer, Int J Neurosci. 91,1997,45).
- Organische Probleme, wie Obstipation (Krygowska-Wajs, Funct Neurol 15,2000,41).
- Depressionen (Camicioli R. Drugs Today (Barc). 2002 Oct;38(10):677-86).
- Kurzfristige Bewegungsanomalien, wie z.B. Chorea oder orthostatische Auffälligkeiten.
- Kombinationen aus oben genannten Markern (Stern, Annals of Neurol 56, 2004, 169).

Dadurch eröffnet sich die Chance, den Krankheitsprozess zu einem Zeitpunkt zu beeinflussen, zu dem noch mehr Neurone vorhanden sind als zum Zeitpunkt des Auftretens mehrerer motorischer Kardinalsymptome der Parkinson'schen Krankheit und damit eine quantitativ höhere Anzahl von Neuronen zu schützen. Eine Gabe eines effektiven Neuroprotektivums zu einem frühen Stadium lässt erwarten, dass der Krankheitsprozess deutlich verzögert werden kann: Je früher eine Therapie einsetzen kann, um so höher sind die Chancen eine langanhaltende Verhinderung des Beginns der die Lebensqualität einschränkenden Symptome zu erreichen.

Es besteht daher der Bedarf an Arzneimitteln, die nicht nur die dopaminerge Übertragung beeinflussen und die Symptomatik der Parkinson'schen Krankheit in fortgeschrittenen Stadien lindern können, sondern die den fortschreitenden Untergang dopaminerger Neuronen in frühen, motorisch weitgehend asymptomatischen Parkinsonstadien umkehren, verhindern oder zumindestens deutlich verlangsamen können (Dawson, Nature Neuroscience Supplement 5, 2002, 1058).

Aus der US 4,564,628, der US 4,885,308, der US 4,722,933 und der WO 01/38321 sind substituierte 2-Aminotetraline bekannt. Es handelt sich um Substanzen mit dopaminerger Wirkung, die zur symptomatischen Behandlung von Morbus Parkinson bekannt sind. Insbesondere das Rotigotin [(-)-5,6,7,8-Tetrahydro-6-[propyl[2-(2-thienyl)ethyl]amino]-1-naphthol] hat sich in klinischen Studien als effektives, transdermal verfügbares Antiparkinsonmittel erwiesen. Die WO 02/089777 beschreibt beispielsweise die transdermale Gabe von Rotigotin bei Parkinson-Patienten und die damit verbundene Verbesserung des UPDRS (Unified Parkinson's Disease Rating Scale)-Scores. Der UPDRS-Score ist ein wichtiges Instrument zur Diagnose und zum Monitoring der Progression bzw. der Therapie von Parkinson-Patienten (Fahn S, Elton RL, Members of the UPDRS Development Committee (1987) Unified Parkinson's Disease Rating Scale. In: Fahn, S, CD Marsden, DB Calne, M Goldstein (eds) Recent Devlopments in Parkinson's Disease. Vol II. Macmillan Healthcare Information, Florham Park (NJ), pp 153-163, 293-304). Allerdings wird mit dem UPDRS-Score lediglich der Effekt eines Wirkstoffs auf die Parkinson-Symptomatik erfasst. Er erlaubt keine Aussagen darüber, ob ein Wirkstoff den der Symptomatik zugrunde liegenden dopaminergen Zelluntergang beeinflusst.

Metman et al (Clin Neuropharmacol 24,2001, 163) beschreiben ebenfalls die Wirkung von Rotigotin auf Parkinson-assoziierte Bewegungsstörungen. Die behandelten Patienten hatten bereits ausgeprägte Dyskinesien, die durch Rotigotin-Gabe verbessert wurden.

Aus dem Stand der Technik sind substituierte 2-Aminotetraline, insbesondere Rotigotin, somit als Dopamin-Agonist zur symptomatischen Behandlung von Morbus Parkinson bekannt. Rein symptomatisch wirkende Parkinson-Arzneimittel versprechen jedoch bei der vorbeugenden Behandlung von Morbus Parkinson keinen Vorteil, da sie keinen Einfluß auf den dopaminergen Zelluntergang und auf das Fortschreiten bzw. die Entstehung der Krankheit haben.

Experimentelle Untersuchungen zeigten nun überraschend, dass die bisher nur zur symptomatischen Therapie der Parkinson'schen Krankheit eingesetzten substituierten 2-Aminotetraline der allgemeinen Formel I 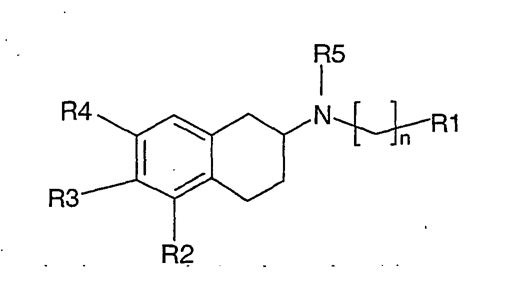 worin gilt:
n ist 1-5;
R2 ist OA; R3 und R4 sind jeweils unabhängig voneinander ausgewählt aus H und OA;
   wobei A ausgewählt ist aus H, Alkyl, Alkoxymethyl oder einer Gruppe 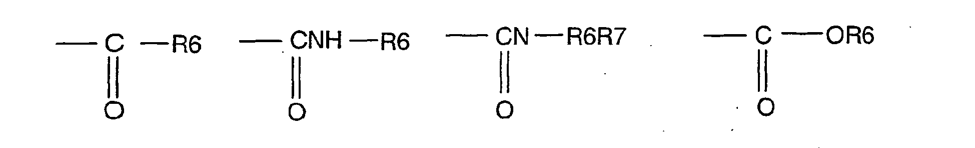 worin R6 und R7 jeweils unabhängig voneinander Alkyl, insbesondere C1-20 Alkyl und besonders bevorzugt C1-6 Alkyl oder Aryl, insbesondere optional substituiertes Phenyl, sind;
R5 ein C1-3 Alkyl ist;
R1 eine Gruppe ist, die ausgewählt ist aus Wasserstoff, 3-Pyridyl, 4-Pyridyl, optional substituiertem Phenyl, 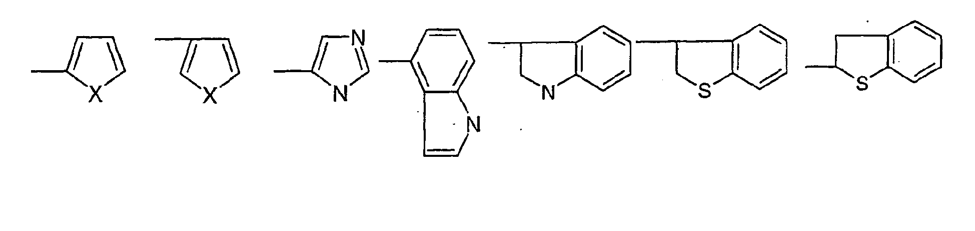
worin X ausgewählt ist aus S, O oder NH;
wobei die Verbindung der Formel I als Razemat oder als reines (R)- oder (S)-Enantiomer vorliegen kann,
sowie physiologisch akzeptable Salze dieser Verbindungen
neuroprotektive Eigenschaften besitzen, so daß diese als Arzneimittel bzw. Prophylaktikum zur Verhinderung des dopaminergen Zellverlusts insbesondere in sehr frühen Stadien der Parkinson'schen Krankheit oder bei Risikokandidaten eingesetzt werden können.

### Abbildungen

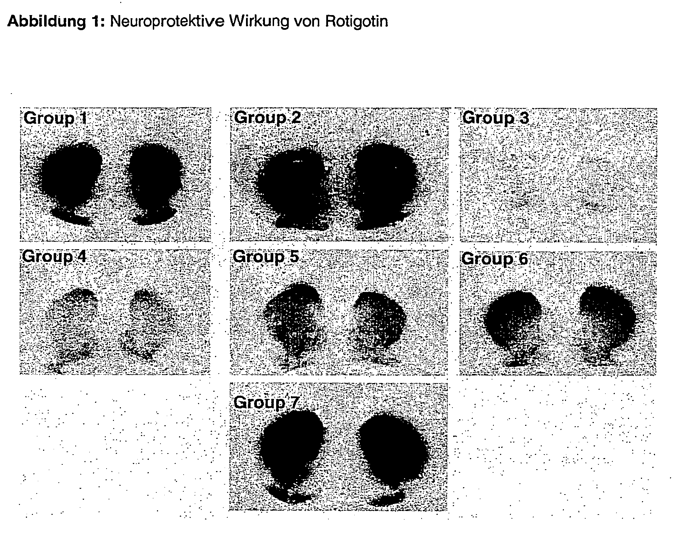

Abbildung 1 zeigt repräsentative Beispiele für die neuroprotektive Wirkung von Rotigotin gemessen an der Dichte der Dopamin-Transporter als Indiz für die Dichte der verbliebenen Nervenendigungen im Striatum

Die Gruppen 1-7 wurden wie folgt behandelt: Gruppe 1: unbehandelte Kontrolle; Gruppe 2: Kontrolle behandelt mit Vehikellösung für Rotigotin und MPTP; Gruppe 3: MPTP-Behandlung; Gruppe 4: MPTP-Behandlung plus Rotigotin 0.3 mg/kg; Gruppe 5: MPTP-Behandlung plus Rotigotin 1.0 mg/kg; Gruppe 6: MPTP-Behandlung plus Rotigotin 3.0 mg/kg; Gruppe 7: Behandlung nur mit Rotigotin (3.0 mg/kg)

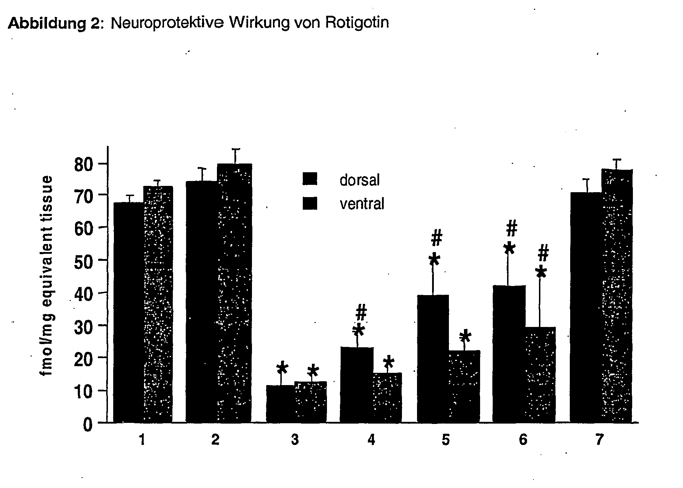

Abbildung 2 zeigt die Dopamin-Transporter (DAT) Bindung im dorsalen und ventralen Teil des Striatums in verschiedenen Gruppen durch Quantifizierung der DAT-Dichte nach einem Experiment wie in Abbildung 1 illustriert. Die Balkendiagramme 1-7 entsprechen den in Abbildung 1 wiedergegebenen Gruppen 1-7. Die mit * gekennzeichneten Gruppen zeigten einen signifikanten Abfall in der DAT Bindung im Vergleich zur Kontrollgruppe 2. Die mit # gekennzeichneten Gruppen wiesen einen signifikanten Erhalt der DAT Bindung im Vergleich mit der MPTP-behandelten Gruppe 3 auf.

### Beschreibung der Erfindung

Apoptotische Vorgänge sollen beim Untergang dopaminerger Neürone in der Parkinson-Pathogenese eine wichtige Rolle spielen (Barzilai, Cell Mol Neurobiol 21, 2001, 215). Es werden daher neuroprotektive Substanzen gewünscht, die den dopaminergen Zelluntergang stoppen oder sogar umkehren können. Als prädiktiv für die geforderten neuroprbtektiven Eigenschaften gilt dabei das MPTP-Modell (Dawson, Nature Neuroscience Supplement 5, 2002, 1058).

Rotigotin zeigt sowohl in einem akuten wie in einem subakuten MPTP-Modell überraschend das gewünschte pharmakologische Profil. Die Untersuchungsergebnisse legen nahe, dass mit Rotigotin apoptotische Prozesse verhindert werden.

Die erfindungsgemäßen 2-Aminotetraline, insbesondere Rotigotin, zeigen dabei neuroprotektive Wirkung in einem Parkinsonmodell der Maus: Nach akuter Gabe von MPTP, das bei Menschen wie bei Affen ein Parkinson-Syndrom erzeugt, wurde zum einen die Anzahl der in der akuten Phase degenerierenden Neurone gemessen (Tabelle 1) und zum anderen die funktionelle Integrität des Striatums in der subaktuen Phase durch Bestimmung der Dichte des Dopamin-Transporters in den terminalen Nervenendigungen erfasst (Abbildungen 1 und 2). In beiden Fällen konnte gezeigt werden, dass Rotigotin neuroprotektiv wirksam war: Zum einen war die Anzahl degenerierender Neurone im Mesenzephalon nach der Gabe von Rotigotin verringert und zum anderen war die dopaminerge Innervation des Striatums nahezu vollständig erhalten bzw. wieder hergestellt.

**Tabelle 1: Anzahl degenerierender Neurone bei der Maus dargestellt mit FluoroJade Färbung**

| Gruppe | Anzahl degen. Neurone | Standart-bweichung |
|---|---|---|
| 1: Vehikel-behandelte Kontrolle | 2.0 | 2.4 |
| 2: MPTP Intoxikation | 73.5 | 34.0 |
| 3: MPTP Intoxikation + Rotigotine 0.3 mg/kg | 66.7 | 30.5 |
| 4: MPTP Intoxikation + Rotigotine 1.0 mg/kg | 76.8 | 41.6 |
| 5: MPTP Intoxikation + Rotigotine 3.0 mg/kg | 34.9 | 31.9 |
| 5: MPTP -Vehikel + Rotigotine 3.0 mg/kg | 3.8 | 4.3 |

In einer Pilotstudie wurde zudem die neuroprotektive Wirkung von Rotigotin an Affen untersucht.

In dem verwendeten Modell, das den progressiven Verlauf der Parkinson'schen Erkrankung an Primaten widerspiegelt, wurden Affen (Makaken) unterschwellig-toxische Dosen von MPTP über mehrere Tage injiziert. Die Parkinson-Symptomatik entwickelte sich in dem Modell über einen Zeitraum von ca 2 Wochen. Sobald ein bestimmter Grad der Schädigung erreicht war, wurden tägliche Injektionen von Rotigotin in einer Formulierung vorgenommen, die einen kontinuierlichen Plasmaspiegel über 24 h erzeugt. Die Injektionen von MPTP wurden gestoppt, sobald die motorische Aktivität um einen bestimmten Grad vermindert war (ca 5 Tage später). Das Verhalten der Tiere wurde täglich bewertet. Sechs Wochen nach Beginn der MPTP-Applikation wurden die Injektionen von Rotigotin gestoppt und die Tiere wurden für weitere zwei Wochen ohne Behandlung beobachtet. Es wurde beobachtet, daß die motorische Aktivität der Tiere unter Behandlung und auch in der folgenden Auswaschphase deutlich verbessert war.

Am Ende der Rotigotin-Applikation bzw. am Ende der Auswaschphase wurde je eine Gruppe von Tieren getötet und der Zustand der Basalganglien histologisch und biochemisch untersucht. Die Dichte der Nervenendigungen im Striatum war gegenüber den unbehandelten Tieren deutlich erhöht. Der Gehalt an Pre-Pro-Enkephalin, ein Indikator für die intakte Vernetzung im 'indirect pathway' der Basalganglien, zeigte nach der Behandlung und nach der Auswaschphase eine Tendenz zur Normalisierung.

Die Resultate zeigen, daß das neuroprotektive Potential von Rotigotin auch in einem Primatenmodel von Parkinson'scher Erkrankung nachgewiesen werden kann. Damit kann eine neuroprotektive Wirkung auch beim Menschen angenommen werden.

Mit Rotigotin und strukturverwandten substituierten 2-Aminotetralinen der allgemeinen Formel I wurden damit der Therapie Wirkstoffe zur Verfügung gestellt, die in idealer Weise zur Herstellung von Arzneimitteln, bzw. Prophylaktika zur Prävention des dopaminergen Neuronenverlustes geeignet sind.

Ein Gegenstand der vorliegenden Anmeldung ist daher die Verwendung von substituierten 2-Aminotetralinen der allgemeinen Formel I, wie weiter unten wiedergegeben, sowie insbesondere von Rotigotin zur Herstellung eines Arzneimittels zur Behandlung oder Prävention des dopaminergen Neuronenverlusts bei Patienten, die an einer neurodegenerativen Erkrankung leiden, die mit einem erhöhten dopaminergen Zelluntergang verbunden ist oder die,ein erhöhtes Risiko eines erhöhten dopaminergen Zelluntergangs aufweisen.

Ein erhöhter dopaminerger Neuronenverlust tritt regelmäßig bei Morbus Parkinson-Patienten auf, wird jedoch auch häufig bei anderen neurodegenerativen Erkrankungen, z.B. bei alpha-Synucleopathien oder der Huntington'schen Erkrankung sowie bei REM-Schlafstörungen und Störungen des Geruchssinns beobachtet.

Im Vergleich zur bisherigen Anwendung der Aminotetraline der Formel I, insbesondere von Rotigotin, die auf die rein symptomatische Behandlung von Morbus Parkinson-Patienten mit Bewegungsstörungen beschränkt war, wird damit als neues Anwendungsgebiet die prophylaktische Behandlung von Individuen erschlossen, die weniger als zwei der Kardinalsymptome der Parkinson' schen Krankheit aufweisen und die somit weniger der symptomatischen, als vielmehr der neuroprotektiven, prophylaktischen Therapie bedürfen. Wie bereits weiter oben beschrieben, profitieren solche Individuen besonders von der neuroprotektiven Wirkung von Rotigotin, da durch die Rotigotin-Gabe der dopaminerge Zellverlust zu einem Zeitpunkt gestoppt oder verlangsamt wird, zu dem noch eine höhere Zahl dopaminerger Neuronen vorhanden ist, als dies bei Patienten der Fall ist, die bereits motorische Symptome aufweisen.

Ein Gegenstand der Erfindung ist daher die Verwendung von substituierten 2-Aminotetralinen der allgemeinen Formel I 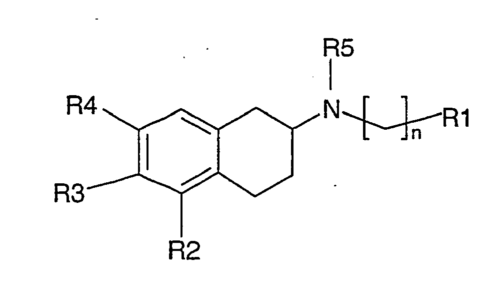 worin gilt:
n ist 1-5;
R2 ist OA; R3 und R4 sind jeweils unabhängig voneinander ausgewählt aus H und OA;
   wobei A ausgewählt ist aus H, Alkyl, Alkoxymethyl oder einer Gruppe 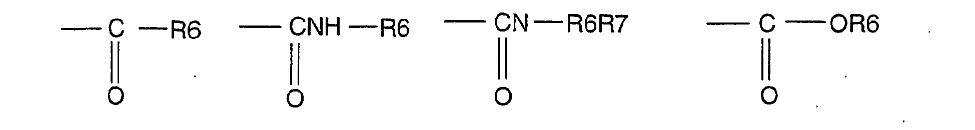 worin R6 und R7 jeweils unabhängig voneinander Alkyl, insbesondere C1-20 Alkyl und besonders bevorzugt C1-6 Alkyl oder Aryl, insbesondere optional substituiertes Phenyl, sind;
R5 ein C1-3 Alkyl ist;
R1 eine Gruppe ist, die ausgewählt ist aus Wasserstoff, 3-Pyridyl, 4-Pyridyl, optional substituiertem Phenyl, 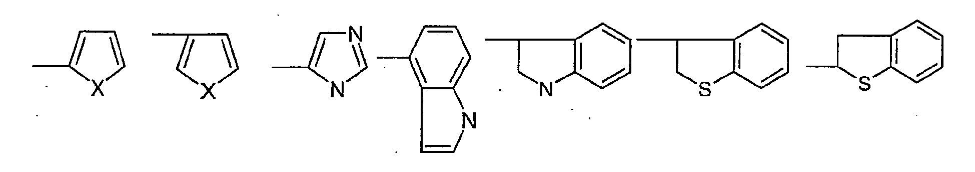
worin X ausgewählt ist aus S, O oder NH;
wobei die Verbindung der Formel I als Razemat oder als reines (R)- oder (S)-Enantiomer vorliegen kann,
sowie physiologisch akzeptable Salze dieser Verbindungen zur vorbeugenden Behandlung von Morbus Parkinson, insbesondere zur Prävention des dopaminergen Zellverlusts von Individuen, bei denen vor Eintritt der vorbeugenden Behandlung wenigstens drei der vier Kardinalsymptome aus der Gruppe Bradykinesie, Rigor, Ruhetremor und posturale Instabilität noch nicht, nur rudimentär oder partiell vorhanden sind.

Zur Herstellung eines Neuroprotektivums bzw. Parkinson-Prophylaktikums besonders geeignete Verbindungen sind solche, in denen R2 eine Gruppe OA ist und R3 und R4 unabhängig voneinander H oder eine Gruppe OA sind, wobei A besonders bevorzugt ein Wasserstoffatom oder eine Gruppe 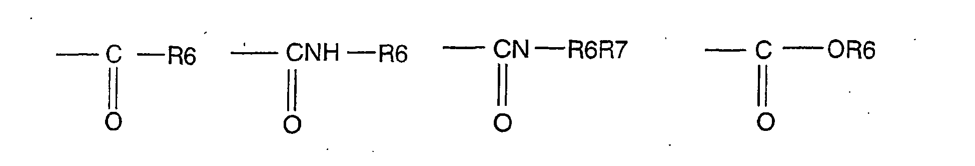 ist, in der R6 ein C1-20 Alkyl, insbesondere C1-12 Alkyl oder C1-6 Alkyl, Phenyl oder Methoxyphenyl ist.

In einer anderen bevorzugten Ausführungsform der Erfindung ist R4 ein H.
In einer anderen bevorzugten Ausführungsform der Erfindung ist R3 ein H.
In einer anderen bevorzugten Ausführungsform der Erfindung sind R3 und R4 beide H.
In einer anderen bevorzugten Ausführungsform der Erfindung ist n= 1, 2 oder 3,
insbesondere n = 2 oder 3

Bevorzugt wird R1 ausgewählt aus der Gruppe H, 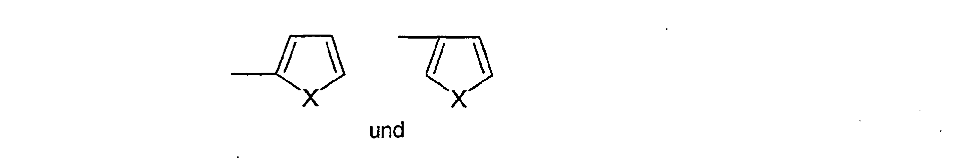 wobei X ausgewählt ist aus S, O und NH und wobei X ganz besonders bevorzugt ein Schwefelatom ist.

Ganz besonders bevorzugt ist R1 2-Thienyl.

In einer weiteren bevorzugten Ausführungsform der Erfindung stellt R5 ein C3-Alkyl, insbesondere n-Propyl dar.

In einer weiteren bevorzugten Ausführungsform der Erfindung ist R1 ein 2-Thienyl, R3 und R4 sind beide H, R5 stellt ein C3-Alkyl dar und n = 2.

In einer besonders bevorzugten Ausführungsform der Erfindung wird zur Herstellung des Parkinson-Prophylaktikums das Razemat von (+/-) 5,6,7,8-Tetrahydro-6-[propyl[2-(2-thienyl)ethyl]amino]-1-naphthol und ganz besonders bevorzugt das reine S-Enantiomer dieser Verbindung (Rotigotin) verwendet.

Unter den Begriffen "C1-20 Alkyl", "C1-12 Alkyl", "C1-3 Alkyl" werden jeweils verzweigte oder unverzweigte Alkylgruppen mit der entsprechenden Zahl C-Atome verstanden. Beispielsweise umfasst ein "C1-20 Alkyl" alle Alkyle mit 1 bis 20 C-Atomen. Die Alkyle können optional substituiert sein, z.B. mit Halogen. Bevorzugt liegen die Alkyle unsubstituiert vor.

Unter dem Begriff "Alkoxymethyl" wird die Gruppe -CH2-O-Alkyl verstanden. Ein bevorzugtes Alkyl ist ein C1-12 Alkyl, ein C1-6 Alkyl oder ein C1-3 Alkyl.

Die prophylaktisch mit den substituierten 2-Aminotetralinen zu behandelnden Individuen können dabei gesund scheinende Individuen sein, deren genetische oder epidemische Prädisposition kein erhöhtes Risiko einer Parkinson'schen Krankheit erkennen lassen.

Für eine Behandlung mit 2-Aminotetralinen, insbesondere Rotigotin, kommen insbesondere Individuen mit erhöhtem Risiko in Frage oder aber Patienten, bei denen klinische, klinisch-chemische oder klinisch-pysikalische Frühsymptome nachweisbar sind, ohne dass diese Patienten jedoch bereits zwei oder mehrere der Kardinalsymptome der Parkinson'schen Krankheit aufweisen.

Schließlich können 2-Aminotetraline, insbesondere Rotigotin als Neuroprotektivum auch dann angewendet werden, wenn die Diagnose nicht eindeutig ist, aber eine Entwicklung einer Symptomatik in Richtung Parkinson-änlicher Neurodegeneration erwarten läßt.

Der Prävention des neuronalen Zellverlusts bedürfen insbesondere
(a) Individuen mit einem erhöhten Risiko für Morbus Parkinson oder
(b) Individuen mit Frühsymptomatik für Morbus Parkinson

Die Begriffe "Morbus Parkinson" und "Parkinson'sche Krankheit" werden in dieser Patentanmeldung als Synonyme verwendet und umfassen idiopathischen und genetischen Parkinson. Davon abzugrenzen ist das sogenannte Parkinson-Plus-Syndrom sowie der sekundare Parkinsonismus.

Unter dem Begriff "Kardinalsymptome" von Morbus Parkinson werden in dieser Patentanmeldung eines oder mehrere der Symptome Bradykinesie, Rigor, Ruhetremor und posturale Instabilität verstanden.

Unter "Individuen mit einem erhöhten Risiko für Morbus Parkinson" werden in dieser Patentanmeldung insbesondere Individuen verstanden, die noch keine nachweisbaren Symptome der Parkinson'schen Krankheit aufweisen, die aber bestimmte Risikofaktoren aufweisen.

Solche Risikofaktoren können genetische Mutationen sein (Nussbaum NEJM 348,2003, 25). Beispielsweise wird das Parkin-Gen auf Chromosome 6q25.2-27 (PARK2) assoziiert mit juvenilem Parkinsonismus und tritt in Familien mit autosomal rezessiver Parkinson-Vererbung verstärkt auf (Matsumine, Am. J. Hum. Genet., 60, 1997, 588; Kitada, Nature 392, 1998, 605; Abbas, Hum. Mol. Genet. 8, 1999, 567; Tassin, Am. J. Hum. Genet., 63, 1998, 88 und Lucking, N. Engl. J. Med. 342, 2000, 1560-7). Weitere Gen-Loci, z.B. PARK6 and PARK7 wurden ebenfalls vermehrt in Familien mit juveniler, rezessiv vererbter Parkinson'schen Krankheit gefunden (Valente, Am. J. Hum. Genet. 68, 2001,895; van Dujin, Am. J. Hum. Genet. 69, 2001, 629). Mutationen im alpha-synuclein-Gen (PARK1) wurden in Familien mit juvenilem, autosomal dominant vererbtem Morbus Parkinson nachgewiesen (Polymeropoulos, Science 276,1997, 2045). Neben genetischen Prädispositionen können auch Umwelteinflüsse, wie die starke Exposition z.B. mit Insektiziden (Vanacore, Neurol Sci., Sep;23 Suppl 2, 2002, S119) Risikofaktoren darstellen.

Unter "Individuen mit Parkinson-Frühsymptomatik" werden in dieser Patentanmeldung insbesondere solche Individuen verstanden, bei denen mindestens drei der vier Kardinalsymptome (Rigor, Ruhetremor, Bradykinesie und posturale Instabilität) noch nicht, nur rudimentär oder partiell vorhanden sind, die aber diagnostisch nutzbare klinische, klinisch-biochemische und/oder klinisch-pysikalische Frühsymptome aufweisen.

Klinisch-biochemische Marker können Veränderungen des alpha-Synuclein oder Neuromelanin-Musters sein. Solche Veränderungen können z.B. beruhen auf der Expression genetischer Varianten z.B. des alpha Synucleins, der Entstehung von Aggregaten oder Filamenten, z.B. des alpha-Synucleins oder der verstärkten Freisetzung aus zellulären Speichern, z.B. aus dem Cytoplasma untergehender Zellen, wie beim Neuromelanin.

Klinisch-physikalische Frühsymptome können strukturelle oder funktionelle Änderungen des Gehirns sein, die sich beispielsweise physikalisch durch PET- und SPECT-Studien, durch transkranielle Sonographie (Becker, J Neurol 249, Suppl 3, 2002,III/40); Prunier C, et al., Neuroimage. 2003 Jul;19(3):810-6) oder durch den Nachweis von biochemischen Markern wie Neuromelanin nachweisen lassen (WO 02/31499).

Klinische Frühsymptome können Riechstörungen, Depressionen, Störungen visueller und kognitiver Funktionen oder Schlafstörungen sein, wobei zur Frühdiagnose auch eine Mischung verschiedener Tests herangezogen werden kann (Becker, J Neurol 249, Suppl 3, 2002, III/40; Stern, Annals of Neurol 56, 2004, 169).

Wie bereits weiter oben diskutiert, sind zum Zeitpunkt des ersten Auftretens von mindestens zwei der vier Kardinalsymptome bereits ca 70-80% der dopaminergen Neuronen der Substantia nigra untergegangen. Um das überraschende neuroprotektive Potenzial der Aminotetraline der Formel I, insbesondere von Rotigotin effektiv zu nutzen, setzt die prophylaktische Behandlung der Patienten daher bevorzugt in einem Stadium ein, in dem die Patienten einen geringeren Verlust dopaminerger Zellen der Substantia Nigra (SN) aufweisen. Bevorzugt werden daher Individuen behandelt, die erst eines oder noch keines der Kardinalsymptome der Parkinson'schen Krankheit in deutlich ausgeprägter Form aufweisen.

Bevorzugt werden Individuen behandelt, die einen dopaminergen Zellverlust in der SN von weniger als 70 %, 60%, 50% und besonders bevorzugt von weniger als 40%, 30%, 20% oder 10% aufweisen.

Als Hilfsmittel zur Diagnose und zur Therapiekontrolle bei motorisch bereits auffälligen Patienten können zwei Scores herangezogen werden: Der UPDRS-Score und der Hoehn und Yahr-Score.

In einem bevorzugten Aspekt der Erfindung weist das prophylaktisch mit den Aminotetralinen der Formel I, insbesondere mit Rotigotin behandelte Patientenkollektiv zudem einen modifizierten Hoehn und Yahr-Score von 0 bis 2, besonders bevorzugt von 0 bis 1 und ganz besonders bevorzugt von 0 auf.

| Tabelle 2: Modifizierte Stadienbestimmung nach Hoehn, The natural history of Parkinson's disease in the pre-levodopa and post-levodopa eras. Neurologic Clinics 10, 1992, 331 |
|---|
| Stadium 0 = Keine Anzeichen der Erkrankung. |
| Stadium 1 = Einseitige Erkrankung. |
| Stadium 1.5 = Einseitige und axiale Beteiligung. |
| Stadium 2 = Beidseitige Erkrankung ohne Gleichgewichtsstörung. |
| Stadium 2.5 = Leichte beidseitige Erkrankung mit Ausgleich beim Zugtest. |
| Stadium 3 = Leichte bis mäßige beidseitige Erkrankung: |
| leichte Haltungsinstabilität; körperlich unabhängig. |
| Stadium 4 = Starke Behinderung; kann noch ohne Hilfe laufen oder stehen. |
| Stadium 5 = Ohne Hilfe an den Rollstuhl gefesselt oder bettlägerig. |

Üblicherweise werden Patienten mit einem UPDRS-Score, Teil III (siehe Ausführungsbeispiel 5) von mindestens 10 als für die dopaminerge Therapie in Frage kommend eingestuft. Das zur Nutzung der neuroprotektiven Wirkung von substituierten 2-Aminotetralinen der Formel I, insbesondere von Rotigotin geeignete Patientenkollektiv weist aber bevorzugt einen sehr niedrigen oder nicht nachweisbaren motorischen UPDRS-Score (Teil III) auf. Im Sinne der vorliegenden Erfindung sollte die vorbeugende Behandlung mit substituierten 2-Aminotetralinen der Formel I, insbesondere mit Rotigotin daher bevorzugt bei Patienten erfolgen, die einen UPDRS Motor-Score von weniger als 10, 9, 8, 7, 6, 5, 4, 3, 2 oder 1 aufweisen. Besonders bevorzugt weisen die Patienten noch keinerlei motorische Störungen auf.

Die Begriffe "Prävention", "Prophylaxe" und "vorbeugende Behandlung" werden in dieser Patentanmeldung als Synonyme verwendet. Sie umfassen insbesondere die Gabe eines Arzneimittels an Individuen, bei denen mindestens drei der vier Kardinalsymptome von Morbus Parkinson (Rigor, Ruhetremor, Bradykinesie, posturale Instabilität) noch nicht, nur rudimentär oder partiell vorhanden sind, um das Auftreten oder die signifikante Ausprägung der motorischen Symptomatik der Parkinson'schen Krankheit und/oder weiteren dopaminergen Neuronenverlust, insbesondere in der Substantia nigra, zu verhindern oder zu verzögern. Bevorzugt weisen die prophylaktisch zu behandelnden Individuen noch keines der Kardinalsymptome in deutlich ausgeprägter Form vor.

Verbindungen der Formeln I sind optisch aktiv und können als Razemate oder als reine (R)- oder (S)-Enantiomere vorliegen. Unter dem Begriff "reines Enantiomer" wird in dieser Patentanmeldung verstanden, dass eine Substanz vorzugsweise zu mindestens 90 Mol% in Form des einen Enantiomer, z.B. der (S)-Form, vorliegt, während der Anteil des jeweils anderen Enantiomers, z.B. der (R)-Form, entsprechend gering ist. Wird zur Herstellung des erfindungsgemäßen Arzneimittels beispielsweise Rotigotin ([(-)-5,6,7,8-Tetrahydro-6-[propyl[2-(2-thienyl)ethyl]amino]-1-naphthol] verwendet, liegt das R(+)-Enantiomer bevorzugt mit einem Anteil von < 10 Mol%, besonders bevorzugt mit einem Anteil von < 2 Mol% und ganz besonders bevorzugt mit einem Molanteil von < 1 % bezogen auf die Gesamtrotigotinmenge im Parkinson-Prophylaktikum vor.

Verbindungen der Formel I können als freie Basen oder in Form der physiologisch akzeptablen Salze, z.B. in Form des Rotigotin-Hydrochlorids, im Arzneimittel vorliegen.

"Physiologisch akzeptable Salze" schließen nicht-toxische Additionssalze einer Verbindung der Formel (I) in Form der freien Base, mit organischen oder anorganischen Säuren ein. Beispiele für anorganische Säuren schließen HCl ein.

Zur Verabreichung von substituierten 2-Aminotetralinen der Formel I, insbesondere von Rotigotin stehen verschiedene Applikationswege zur Verfügung, die der Fachmann je nach Bedarf, Zustand und Alter des Patienten, erforderlicher Dosierung und gewünschtem Applikationsintervall auswählen und anpassen kann.

Eine bevorzugte Art der Verabreichung von substituierten 2-Aminotetralinen der Formel I, insbesondere von Rotigotin ist die transdermale Gabe. Die Darreichungsform kann grundsätzlich ausgewählt sein aus z.B. Salbe, Paste, Spray, Folie, Pflaster oder einer iontophoretischen Vorrichtung.

Bevorzugt werden substituierte 2-Aminotetraline der Formel I, insbesondere Rotigotin dabei in Pflasterform auf die Haut des Patienten gebracht, wobei der Wirkstoff bevorzugt in einer Matrix aus adhesivem Polymer, z.B. einem selbstklebendem adhesivem Polysiloxan, vorliegt. Beispiele für geeignete transdermale Formulierungen finden sich in WO 99/49852, WO 02/89777 und WO 02/89778. Eine solche Darreichungsform ermöglicht die Einstellung eines weitgehend konstanten Plasmaspiegels und damit eine konstante dopaminerge Stimulation über das gesamte Applikationsintervall (WO 02/89778; Metman, Clinical Neuropharmacol. 24, 2001, 163).

Wird dagegen ein Arzneimittel in Form einer subkutanen oder intramuskularen Depotform gewünscht, können substituierte 2-Aminotetraline der Formel I, insbesondere Rotigotin beispielsweise als Salzkristalle, z.B. als kristallines Rotigotin-Hydrochlorid, in einem hydrophobem, wasserfreiem Medium suspendiert und injiziert werden, wie in WO 02/15903 beschrieben oder auch in Form von Mikrokapseln, Mikropartikeln oder Implantaten auf Basis bioabbaubarer Polymere, wie beispielsweise in WO 02/38646 beschrieben, verabreicht werden.

Andere denkbare Formen der Verabreichung von substituierten 2-Aminotetralinen der Formel I, insbesondere von Rotigotin sind transmukosale Formulierungen, z.B. Sublingualsprays, rektale Formulierungen oder Aerosole zur pulmonalen Verabreichung.

Geeignete Dosierungen von substituierten 2-Aminotetralinen der Formel I, insbesondere von Rotigotin liegen zwischen 0,05 und ca. 50 mg/Tag, wobei vorzugsweise Tagesdosen zwischen 0,1 und 40 mg und insbesondere zwischen 0,2 und 20 mg/Tag verabreicht werden. Dabei kann die Dosierung einschleichend erfolgen, das heißt, die Behandlung kann gegebenenfalls mit niedrigen Dosierungen beginnen, die dann bis zur Erhaltungsdosis gesteigert werden.

Dem Fachmann ist klar, dass das Dosierungsintervall in Abhängigkeit von der applizierten Menge, der Applikationsart und dem Tagesbedarf des Patienten variieren kann. So kann eine transdermale Applikationsform beispielsweise zur einmal täglichen, dreitägigen oder siebentägigen Verabreichung konzipiert sein, während ein subkutanes oder intramuskuläres Depot Injektionen beispielsweise im Ein-, Zwei- oder Vierwochen-Rhythmus ermöglichen kann.

In der neuroprotektiven Arzneiform können neben substituierten 2-Aminotetralinen der Formel I, insbesondere neben Rotigotin, noch andere Wirkstoffe vorliegen, die die Progression des dopaminergen Zellverlusts verhindern.

Beispiele hierfür sind antiapoptotisch wirksame Substanzen (Minocycline, FK-506, Cyclosporin A, zVAD) sowie Neurotrophine, wie z.B. der Glial-cell-derived neurotrophic factor (GDNF).

In einem Kombinationspräparat kann eine sequentielle Gabe beispielsweise erreicht werden, indem eine Darreichungsform, z.B. eine orale Tablette, zwei unterschiedliche Schichten mit differierendem Freisetzungsprofil für die verschiedenen pharmazeutisch aktiven Bestandteile aufweist. Dem Fachmann ist klar, dass im Kontext der vorliegenden Erfindung verschiedene Darreichungsformen und Applikationsschemata denkbar sind, die alle Gegenstand der Erfindung sind.

Ein weiterer Gegenstand der Anmeldung ist ein Kit zur Frühdiagnose und Behandlung von Morbus Parkinson. Ein solches Kit enthält (a) ein Diagnostikum, dass die Diagnose von Morbus-Parkinson bzw. der Veranlagung zur Erkrankung an Morbus Parkinson in einem frühen oder asymptomatischen Stadium ermöglicht sowie (b) eine pharmazeutische Formulierung umfassend substituierte 2-Aminotetraline der allgemeinen Formel I, insbesondere Rotigotin.

Beispielsweise kann ein solches Kit umfassen:
(a) ein Mittel oder Diagnosekit zum Nachweis von Neuromelanin
(b) eine pharmazeutische Formulierung enthaltend substituierte 2-Aminotetraline der allgemeinen Formel I, insbesondere Rotigotin.

In einer anderen Ausführunsgform der Erfindung kann das Kit enthalten:
(a) ein Mittel oder ein Diagnosekit zum Nachweis von Semaphorin 3
(b) eine pharmazeutische Formulierung enthaltend substituierte 2-Aminotetraline der allgemeinen Formel I, insbesondere Rotigotin.

In einer anderen Ausführunsgform der Erfindung kann das Kit enthalten:
(c) ein Mittel oder ein Diagnosekit zum Nachweis von alpha-Synuclein und/oder seinen Aggregaten
(d) eine pharmazeutische Formulierung enthaltend substituierte 2-Aminotetraline der allgemeinen Formel I, insbesondere Rotigotin.

In einer weiteren Ausführungsform der Erfindung kann das Kit enthalten:
(a) ein Mittel oder ein Diagnosekit zum genetischen Nachweis einer mit dem Auftreten von Morbus-Parkinson verbundenen Mutation und/oder eines mit dem gehäuften Auftreten von Morbus Parkinson assoziierten Allels, insbesondere aus der Gruppe der PARK-Gene 1,2,3,4,5,6,7, oder 8 sowie der CYP2D6-B und GSTM1-Genloci
(b) eine pharmazeutische Formulierung enthaltend substituierte 2-Aminotetraline der allgemeinen Formel I, insbesondere Rotigotin.

### Ausführungsbeispiele:

### Ausführungsbeispiel 1 : Rotigotin-Pflaster

1.8 g Rotigotin (freie Base) werden in 2.4 g Ethanol gelöst und zu 0.4 g Kollidon 90F (gelöst in 1 g Ethanol) gegeben. Diese Mischung wird zu einer 74%igen Lösung von Silikonpolymeren (8.9 g BioPSA 7-4201 + 8.9 g BIO-PSA 7-4301 [Dow Corning]) in Heptan gegeben. Nach Zugabe von 2.65 g Petrolether wird die Mischung für 1 Stunde bei 700 UpM gerührt um eine homogene Dispersion zu erhalten. Nach Laminierung auf Polyester wurde bei 50°C getrocknet. Das Pflastergewicht betrug schließlich 50 g/cm2.

### Ausführungsbeispiel 2: Rotigotin-Depotsuspensionen

(a) 1411,2 g Miglyol 812 wurde in eine Duran Flasche eingewogen. 14,4 g Imwitor 312 wurde dem Miglyol zugegeben und im Anschluß für 30 Minuten unter Rühren auf 80°C erwärmt. Die klare Lösung wurde auf Raumtemperatur abgekühlt und gefiltert.
(b) 1188 g der unter (a) hergestellten Lösung wurde in einen Glaslaborreaktor überführt, 12 g N-0923 zugesetzt und für 10 Minuten mit einem Ultraturrax bei 10.000 UpM unter Stickstoff homogenisiert. Die Suspension wurde bei laufendem Ultraturrax (2.000 UpM) in Braunglasflaschen abgefüllt.

### 3. Ausführungsbeispiel: Subakutes MPTP-Modell

Zur Intoxikation werden Mäusen 80 mg/kg des Neurotoxins 1-Methyl-4-phenyl-1,2,3,6-tetrahydro-pyridine (MPTP) verabreicht (in Portionen von 20 mg/kg in zweistündigen Abständen, Gruppe 3-6 in Abbildung 1 und 2), was dazu führt, dass ca. 50 - 60 % der Neurone der Substantia nigra degenerieren (maximale Degeneration in Gruppe 3 in Abbildung 1 und 2). Rotigotin wird täglich in Dosen zu je 0.3, 1 oder 3 mg/kg über 7 Tage als sogenannte ,Slow-Release-Formulierung' (siehe Ausführungsbeispiel 2) verabreicht (Gruppe 4-6 in Abbildung 1 und 2). Eine Gruppe MPTP-behandelter Tiere (Gruppe 3) erhält Rotigotin-Vehikel-Lösung (siehe Ausführungsbeispiel 2 ohne Rotigotin HCl) und dient als Referenz. Als Kontrollen dienen die Gruppen 1, 2 und 7, wobei Gruppe 1 keinerlei Behandlung erfährt, Gruppe 2 mit den Vehikel-Lösungen für MPTP und Rotigotin behandelt wird und Gruppe 7 ausschließlich Rotigotin erhält. Am 8. Tag werden die Tiere getötet, die Gehirne entnommen und eingefroren. Gefrierschnitte werden mit 100 pm [¹²⁵I] PE2l ([¹²⁵I]-(E)-N(3-iodoprop-2-enyl)-2(3-carboxymethyl-3(3-(4'-methylphenyl)-nortropane) in Phosphat Puffer, pH 7.4, inkubiert, um die Menge der im Striatum noch vorhandenen Dopamin-Transporter zu markieren, was als Indiz für die Menge funktionierender Nervenendigungen dient. Rotigotin verbessert das Überleben der Neurone und ihrer Nervenendigungen dosisabhängig. Dies ist ein klarer Hinweise für die neuroprotektiven Eigenschaften der Substanz (Abbildung 1 und 2).

### 4. Ausführungsbeispiel: Akutes MPTP-Modell (einschließlich Apoptose)

Zur Intoxikation werden Mäusen 80 mg/kg des Neurotoxins 1-methyl-4-phenyl-1,2,3,6-tetrahydro-pyridine (MPTP) verabreicht (in Portionen von 20 mg/kg in zweistündigen Abständen), was dazu führt, dass ca. 50 - 60 % der Neurone der Substantia nigra degenerieren. Ca 16. Stunden vorher wird Rotigotine in Dosen zu je 0.3, 1 oder 3 mg/kg als sogenannte ,Slow-Release-Formulierung' verabreicht. Diffusions- und Absorptionslatenzen führen dazu, daß Rotigotine dann optimal verfügbar ist, wenn MPTP gegeben wird. Nach 24 Stunden werden die Tiere getötet und die Gehirne fixiert. Die Gehirnschnitte werden mit FluoroJade zur Identifzierung degenerierender Zellen gefärbt. Die immunhistochemische Markierung der Tyrosin-Hydroxylase dient der Identifizierung dopaminerger Neurone. Die Färbung der Tyrosin-Hydroxylase ergibt keine Unterschiede zwischen behandelten und unbehandelten Tieren; die Färbung mit FluoroJade zeigt eine große Zahl degenerierender Neurone; die Neurone sind allerdings noch nicht vollständig entfernt; dies legt nahe, dass der Zelluntergang apoptotisch verläuft. Die Anzahl der degenerierenden Neurone ist um ca. 50 % geringer nach Applikation von Rotigotin, was die neuroprotektive Eigenschaft der Substanz weiter belegt (Tabelle 1).

### 5. Ausführungsbeispiel: Bestimmung des motorischen UPDRS-Scores

Der motorische UPDRS Score (Teil III des UPDRS-Scores) wird durch Untersuchung der Patienten anhand der in der folgenden Tabelle 2 wiedergegebenen Kriterien 18-31 bestimmt, wobei die sich aus den jeweiligen Kriterien ergebenden Punktwerte jeweils aufaddiert werden.

**Tabelle 2:**

| **III. MOTORISCHE UNTERSUCHUNG** |
|---|
| **18. Sprache:** |
| □ 0 - Normal. |
| □ 1 - Leichte Abnahme von Ausdruck, Diktion und/oder Volumen. |
| □ 2 - Monoton, verwaschen, aber verständlich; mäßig behindert. |
| □ 3 - Deutliche Beeinträchtigung, schwer zu verstehen. |
| □ 4 - Unverständlich. |

| **19. Gesichtsausdruck:** |
|---|
| □ 0 - Normal. |
| □ 1 - Minimal veränderte Mimik, könnte ein normales " Pokergesicht" sein. |
| □ 2 - Leichte, aber eindeutig abnorme Verminderung des Gesichtsausdruckes. |
| □ 3 - Mäßig verminderte Mimik; Lippen zeitweise geöffnet. |
| □ 4 - Maskenhaftes oder erstarrtes Gesicht mit stark oder völlig fehlendem Ausdruck; Lippen stehen um 7 mm auseinander. |

| **20. Ruhetremor: (G = Gesicht, RH = rechte Hand, LH = linke Hand, RF = rechter Fuß, LF = linker Fuß)** |
|---|
| G RH LH RF LF |
| □ □ □ □ □ 0 - Keine. |
| □ □ □ □ □ 1 - Leicht und selten vorhanden. |
| □ □ □ □ □ 2 - Geringe Amplitude persistierend; oder mäßige Amplitude, aber nur intermittierend auftretend. |
| □ □ □ □ □ 3 - Mäßige Amplitude, die meiste Zeit vorhanden. |
| □ □ □ □ □ 4 - Ausgeprägte Amplitude, die meiste Zeit vorhanden. |

| **21. Aktions- oder Haltungstremor der Hände:** (R = rechts, L = links) |
|---|
| R L |
| □ □ 0 - Fehlt. |
| □ □ 1 - Leicht; bei Bewegung vorhanden: |
| □ □ 2 - Mäßige Amplitude, bei Bewegung vorhanden. |
| □ □ 3 - Mäßige Amplitude, bei Beibehalten der Haltung und bei Bewegung vorhanden. |
| □ □ 4 - Ausgeprägte Amplitude; beim Essen störend. |

| **22. Rigidität: (Geprüft bei passiver Bewegung der großen Gelenke am sitzenden Patienten. Zahnradphänomen kann ignoriert werden). (N = Nacken, ROE = rechte obere Extremität, LOE = linke obere Extremität, RUE = rechte untere Extremität, LUE = linke untere Extremität)** |
|---|
| N ROE LOE RUE LUE |
| □ □ □ □ □ 0- Fehlt. |
| □ □ □ □ □ 1 - Leicht oder nur erkennbar bei Aktivierung durch spiegelbildliche oder andere Bewegungen. |
| □ □ □ □ □ 2 - Leicht bis mäßig. |
| □ □ □ □ □ 3 - Ausgeprägt, jedoch voller Bewegungsumfang bleibt erreicht. |
| □ □ □ □ □ 4 - Stark; Schwierigkeit beim Ausführen aller Bewegungen. |

| **23. Fingerklopfen: (Patient berührt in rascher Reihenfolge und bei größtmöglicher Amplitude und mit jeder Hand gesondert den Daumen mit dem Zeigefinger). (R = rechts, L = links).** |
|---|
| RL |
| □ □ 0 - Normal. |
| □ □ 1 - Leichte Verlangsamung und/oder Verringerung der Amplitude. |
| □ □ 2 - Mäßig eingeschränkt. Eindeutige und frühzeitige Ermüdung. Bewegung kann gelegentlich unterbrochen werden. |
| □ □ 3 - Stark eingeschränkt. Verzögerter Start der Bewegungen oder Unterbrechung fortlaufender Bewegungen. |
| □ □ 4 - Kann die Aufgabe kaum ausführen. |

| **24. Handbewegungen: (Patient öffnet und schließt die Hände in rascher Reihenfolge bei größtmöglicher Amplitude und mit jeder Hand gesondert). (R = rechts, L = links)** |
|---|
| PL |
| □ □ 0 - Normal. |
| □ □ 1 - Leichte Verlangsamung und/oder Verringerung der Amplitude. |
| □ □ 2 - Mäßig eingeschränkt. Eindeutige und frühzeitige Ermüdung. Bewegung kann gelegentlich unterbrochen werden. |
| □ □ 3 - Stark eingeschränkt. Verzögerter Start der Bewegungen oder Unterbrechung fortlaufender Bewegungen. |
| □ □ 4 - Kann die Aufgabe kaum ausführen. |

| **25. Rasch wechselnde Bewegungen der Hände: (Pronation-Supinationsbewegungen der Hände, vertikal oder horizontal, mit größtmöglicher Amplitude, beide Hände gleichzeitig).** |
|---|
| RL |
| □ □ 0 - Normal. |
| □ □ 1 - Leichte Verlangsamung und/oder Verringerung der Amplitude. |
| □ □ 2 - Mäßig eingeschränkt. Eindeutige und frühzeitige Ermüdung. Bewegung kann gelegentlich unterbrochen werden. |
| □ □ 3 - Stark eingeschränkt. Verzögerter Start der Bewegungen oder Unterbrechung fortlaufender Bewegungen. |
| □ □ 4 - Kann die Aufgabe kaum ausführen. |

| **26. Agilität der Beine: (Der Patient klopft in rascher Reihenfolge mit der Ferse auf den Boden und hebt dabei das ganze Bein an. Die Amplitude soll mindestens 7,5 cm betragen.** |
|---|
| RL |
| □ □ 0 - Normal. |
| □ □1 - Leichte Verlangsamung und/oder Verringerung der Amplitude. |
| □ □ 2 - Mäßig eingeschränkt. Eindeutige und frühzeitige Ermüdung. Bewegung kann gelegentlich unterbrochen werden. |
| □ □ 3 - Stark eingeschränkt. Verzögerter Start der Bewegungen oder Unterbrechung fortlaufender Bewegungen. |
| □ □ 4 - Kann die Aufgabe kaum ausführen. |

| **27. Aufstehen vom Stuhl: (Patient versucht mit vor der Brust verschränkten Armen von einem geradelehnigen Holz- oder Metallstuhl aufzustehen).** D 0 - Normal. |
|---|
| □ 1 - Langsam; kann mehr als einen Versuch benötigen. |
| □ 2 - Stößt sich an den Armlehnen hoch. |
| □ 3 - Neigt zum Zurückfallen und muß es eventuell mehrmals versuchen, kann jedoch ohne Hilfe aufstehen. |
| □ 4 - Kann ohne Hilfe nicht aufstehen. |

| **28. Haltung:** |
|---|
| □ 0 - Normal aufrecht. |
| □ 1 - Nicht ganz aufrecht, leicht vorgebeugte Haltung; könnte bei einem älteren Menschen normal sein. |
| □ 2 - Mäßig vorgebeugte Haltung; eindeutig abnorm, kann leicht zu einer Seite geneigt sein. |
| □ 3 - Stark vorgebeugte Haltung mit Kyphose; kann mäßig zu einer Seite geneigt sein. |
| □ 4 - Ausgeprägte Beugung mit extrem abnormer Haltung. |

| **29. Gang:** |
|---|
| □ 0 - Normal. |
| □ 1 - Geht langsam, kann einige kurze Schritte schlurfen, jedoch keine Festination oder Propulsion. |
| □ 2 - Gehen schwierig, benötigt aber wenig oder keine Hilfe; eventuell leichtes Trippeln, kurze Schritte oder Propulsion. |
| □ 3 - Starke Gehstörung, benötigt Hilfe. |
| □ 4 - Kann überhaupt nicht gehen, auch nicht mit Hilfe. |

| **30. Haltungsstabilität: (Reaktion auf plötzliches Verlagern nach hinten durch Ziehen an den Schultern des Patienten; der mit geöffneten Augen und leicht auseinanderstehenden Füßen geradesteht. Der Patient ist darauf** vorbereitet). |
|---|
| □ 0 - Normal. |
| □ 1 - Retropulsion, gleicht aber ohne Hilfe aus. |
| □ 2 - Fehlen einer Haltungsreaktion; würde fallen, wenn er nicht vom Untersucher aufgefangen würde. |
| □ 3 - Sehr instabil; neigt dazu, spontan das Gleichgewicht zu verlieren. |
| □ 4 - Kann nicht ohne Unterstützung stehen. |

| **31. Bradykinesie und Hypokinesie des Körpers: (Kombination aus Langsamkeit, Zögern, verminderten Mitbewegungen der Arme, geringe Bewegungsamplitude und allgemeine Bewegungsarmut)** |
|---|
| □ 0 - Keine. |
| □ 1 - Minimale Verlangsamung, Bewegung wirkt beabsichtigt; könnte bei manchen Menschen normal sein. Möglicherweise herabgesetzte Amplitude. |
| □ 2 - Leichte Verlangsamung und Bewegungsarmut, die eindeutig abnorm sind. Alternativ auch herabgesetzte Amplitude. |
| □ 3 - Mäßige Verlangsamung und Bewegungsarmut oder Herabsetzung der Amplitude. |
| D 4 - Ausgeprägte Verlangsamung, Bewegungsarmut oder Herabsetzung der Amplitude. |

### Ausführungsbeispiel 6: In vitro Umsetzung eines Prodrugs in die Wirksubstanz

Aus Leberzellhomogenaten von Mensch, Affe, Hund, Ratte oder Maus wird durch differentielle Zentrifugation die Mikrosomenfraktion erhalten, die die wesentlichen metabolisierenden Enzyme enthält; alternativ kann auch die zytoplasmatische Fraktion gewonnen werden. Die subzelluläre Fraktion wird mit einem Puffer so suspendiert, dass eine Lösung mit einem definierten Proteingehalt erhalten wird. Nach Zufügen von 1 µM des zu testenden Prodrugs erfolgt die Inkubation bei 37 °C für 60 min. Anschließend wird Rotigotin mittels HPLC/UV oder auch mittels HPLC/MS quantifiziert und zur eingesetzten Menge in Beziehung gesetzt. Für detailliertere Analysen werden Konzentrations- oder Zeitreihen untersucht.

## Patentansprüche

1. Verwendung einer Verbindung der allgemeinen Formel I 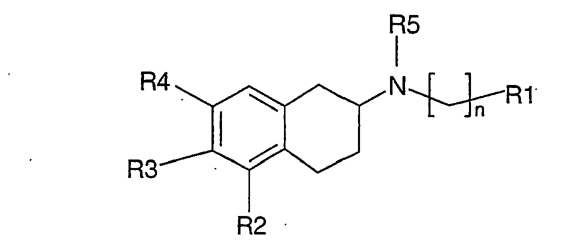 worin gilt:
n= 1-5;
R2 ist = OA; R3 und R4 sind jeweils unabhängig voneinander ausgewählt aus H und OA;
wobei A ausgewählt ist aus H, Alkyl, Alkoxymethyl oder einer Gruppe, 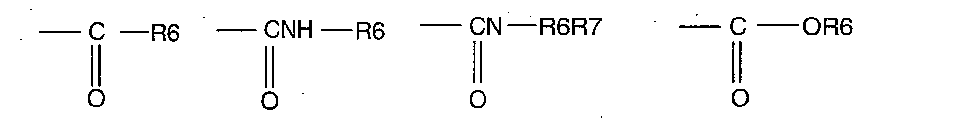 in der R6 und R7 unabhängig voneinander Alkyl oder Aryl sind;
R5 ist ein C1-3 Alkyl;
R1 ist eine Gruppe, die ausgewählt ist aus Wasserstoff, 3-Pyridyl, 4-Pyridyl, optional substituiertem Phenyl, 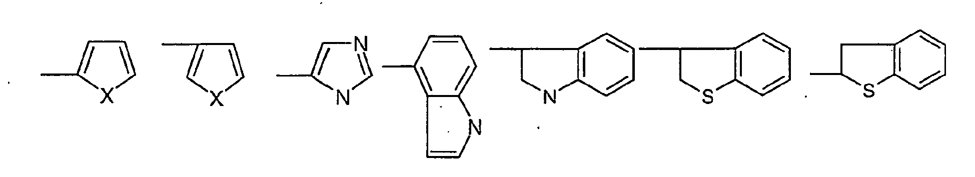
worin X ausgewählt ist aus S, O oder NH;
wobei die Verbindung der Formel I als Razemat oder als reines (R)- oder (S)-Enantiomer vorliegt;
sowie physiologisch akzeptable Salze dieser Verbindungen zur Herstellung eines Arzneimittels zur vorbeugenden Behandlung von Morbus Parkinson.

2. Verwendung nach Anspruch 1, wobei die vorbeugende Behandlung an Individuen erfolgt,
die ausgewählt sind aus der Gruppe
(a) von Individuen ohne Parkinson-Symptome, aber mit einem erhöhten Risiko für Morbus Parkinson oder
(b) von Individuen mit Parkinson-Frühsymptomatik, bei denen mindestens drei der vier Kardinalsymptome von Morbus Parkinson (Rigor, Ruhetremor, Bradykinesie, posturale Instabilität) noch nicht oder nur partiell vorhanden sind.

3. Verwendung nach Anspruch 2, wobei den Individuen unter (b) mehrere der folgenden klinischen Symptome zugeordnet werden können: Riechstörungen, Depressionen, Schlafstörungen vom Typ der ,REM Behavior Disorder', Obstipationen und kurzfristige Bewegungsanomalien.

4. Verwendung nach Anspruch 2, wobei die Individuen eine Mutation in einem PARK-Gen und/oder Veränderungen des alpha-Synuclein oder Neuromelanin-Musters aufweisen.

5. Verwendung nach einem der vorhergehenden Ansprüche, wobei R3 und R4 jeweils Wasserstoff repräsentieren.

6. Verwendung nach einem der vorhergehenden Ansprüche, worin A ein Wasserstoffatom oder eine Gruppe ausgewählt aus 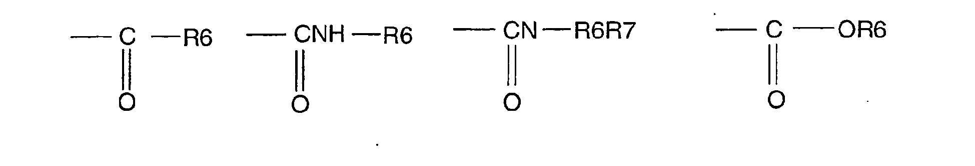 ist, in der R6 C1-12 Alkyl, Phenyl oder Methoxyphenyl ist.

7. Verwendung nach einem der vorhergehenden Ansprüche, wobei n = 1-3.

8. Verwendung nach einem der vorhergehenden Ansprüche, wobei R1 ausgewählt ist aus der Gruppe 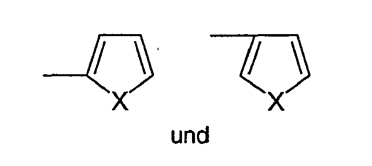 worin X für S, O oder NH steht.

9. Verwendung nach einem der vorhergehenden Ansprüche, wobei X ein Schwefelatom ist

10. Verwendung nach einem der vorhergehenden Ansprüche, wobei R5 ein C3-Alkyl darstellt.

11. Verwendung nach einem der vorhergehenden Ansprüche, wobei R1 ein 2-Thienyl ist, R3 und R4 beide H sind, R5 ein C3-Alkyl darstellt und n=2 ist.

12. Verwendung nach einem der vorhergehenden Ansprüche, wobei die Verbindung 5,6,7,8-Tetrahydro-6-[propyl[2-(2-thienyl)ethyl]amino]-1-naphthol ist.

13. Verwendung nach Anspruch 12, wobei die Verbindung das reine S-Enantiomer (Rotigotin) ist.

14. Verwendung nach einem der vorhergehenden Ansprüche, wobei die Individuen vor Beginn der Arzneimittelgabe einen Verlust dopaminerger Zellen in der Substantia nigra von weniger als 60% aufweisen.

15. Verwendung nach einem der vorhergehenden Ansprüche, wobei die Individuen vor Beginn der Arzneimittelgabe einen UPDRS-Score von weniger als 10 aufweisen.

16. Verwendung nach einem der vorhergehenden Ansprüche, wobei die Individuen einen Höhn-Yahr-Score von 0 oder 1 aufweisen.

17. Verwendung nach einem der vorhergehenden Ansprüche, wobei das Arzneimittel zur parenteralen, transdermalen oder mukosalen Administration vorgesehen ist.

18. Verwendung nach einem der vorhergehenden Ansprüche, wobei die Verbindung der allgemeinen Formel I in einer Dosierung von 0,05 - 50 mg pro Tag verabreicht wird.

19. Kit zur Diagnose und Behandlung von Morbus Parkinson umfassend
(a) ein Diagnostikum, dass die Diagnose von Morbus-Parkinson bzw. der Veranlagung zur Erkrankung an Morbus Parkinson in einem frühen oder asymptomatischen Stadium ermöglicht und
(b) eine pharmazeutische Formulierung umfassend substituierte 2-Aminotetraline der allgemeinen Formel I, wie in einem der Ansprüche 1-13 definiert.

20. Kit nach Anspruch 19, wobei das Diagnostikum (a) ausgewählt ist aus:
(i) einem Mittel oder Diagnosekit zum Nachweis von Neuromelanin
(ii) einem Mittel oder Diagnosekit zum Nachweis von Semaphorin 3
(iii) einem Mittel oder Diagnosekit zum Nachweis von alpha-Synuclein und/oder seinen Aggregaten oder
(iv) einem Mittel oder Diagnosekit zum genetischen Nachweis einer mit dem Auftreten von Morbus-Parkinson verbundenen Mutation und/oder eines mit dem gehäuften Auftreten von Morbus Parkinson assoziierten Allel

## Claims

1. Use of a compound of the general formula (I): 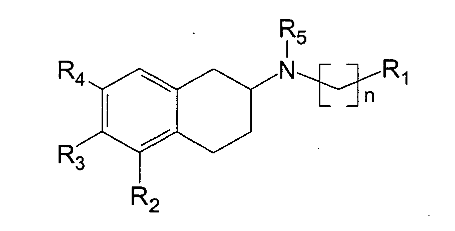 wherein:
n = 1 to 5;
R₂ is OA; R₃ and R₄ are each independently selected from H and OA; wherein A is selected from H, alkyl, alkoxymethyl or a group 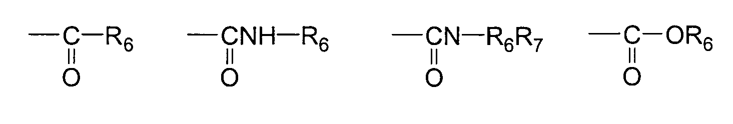 wherein R₆ and R₇ are independently alkyl or aryl;
R₅ is a C₁₋₃-alkyl;
R₁ is a group selected from hydrogen, 3-pyridyl, 4-pyridyl, optionally substituted phenyl, 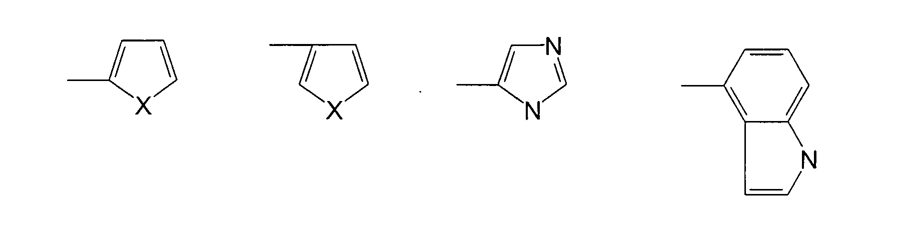 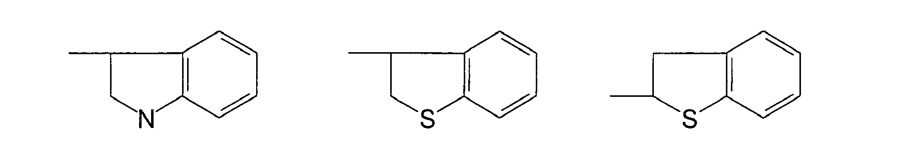
wherein X is selected from S, O or NH;
wherein the compound of formula (I) is present as a racemate or as a pure (R)- or (S)-enantiomer;
as well as physiologically acceptable salts of these compounds for the preparation of a medicament for the preventative treatment of Parkinson's disease.

2. Use according to claim 1, wherein the preventative treatment is performed on individuals, selected from the group of
(a) individuals without symptoms of Parkinson's disease but with an increased risk of developing Parkinson's disease, or
(b) individuals with early symptoms of Parkinson's disease in whom at least three of the four cardinal symptoms of Parkinson's disease (rigidity, resting tremors, bradykinesia, postural instability) are not yet or are only partially present.

3. Use according to claim 2, wherein the individuals defined in point (b) display several of the following clinical symptoms: olfactory disorders, depressions, sleeping disorders of the type "REM Behavior Disorder", constipation and short-term movement anomalies.

4. Use according to claim 2, wherein the individuals have a mutation in a PARK-gene and/or modifications to the alpha-synuclein or neuromelanine pattern.

5. Use according to one of the preceding claims, wherein R₃ and R₄ each represent hydrogen.

6. Use according to one of the preceding claims, wherein A is a hydrogen atom or a group selected from 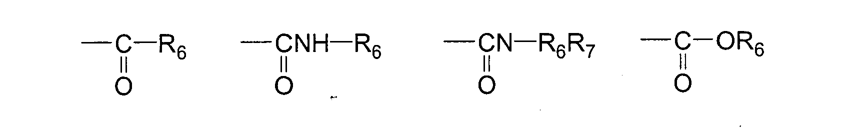 wherein R₆ is C₁₋₁₂-alkyl, phenyl or methoxyphenyl.

7. Use according to one of the preceding claims, wherein n = 1 to 3.

8. Use according to one of the preceding claims, wherein R₁ is selected from the group 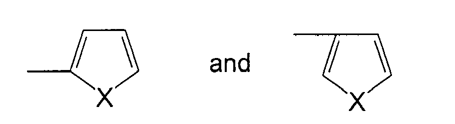 wherein X is S, O or NH.

9. Use according to one of the preceding claims, wherein X is a sulphur atom.

10. Use according to one of the preceding claims, wherein R₅ is C₃-alkyl.

11. Use according to one of the preceding claims, wherein R₁ is a 2-thienyl, R₃ and R₄ are both H, R₅ is a C₃-alkyl and n = 2.

12. Use according to one of the preceding claims, wherein the compound is 5,6,7,8-tetrahydro-6-[propyl[2-(2-thienyl)ethyl]amino]-1-naphthol.

13. Use according to claim 12, wherein the compound is the pure S-enantiomer (rotigotine).

14. Use according to one of the preceding claims, wherein the individuals have a dopaminergic cell loss in the Substantia nigra of less than 60 % before commencement of medicament administration.

15. Use according to one of the preceding claims, wherein the individuals have a UPDRS score of less than 10 before commencement of medicament administration.

16. Use according to one of the preceding claims, wherein the individuals have a Höhn-Yahr score of 0 or 1.

17. Use according to one of the preceding claims, wherein the medicament is provided for parenteral, transdermal or mucosal administration.

18. Use according to one of the preceding claims, wherein the compound of general formula (I) is administered in a dose of 0.05 to 50 mg per day.

19. Kit for the diagnosis and treatment of Parkinson's disease, comprising
(a) a diagnostic agent that enables the diagnosis of Parkinson's disease and/or the disposition to develop Parkinson's disease at an early or asymptomatic stage, and
(b) a pharmaceutical formulation, comprising substituted 2-aminotetralines of general formula (I), as defined in one of claims 1 to 13.

20. Kit according to claim 19, wherein the diagnostic agent (a) is selected from:
(i) an agent or a diagnosis kit for detecting neuromelanine
(ii) an agent or a diagnosis kit for detecting Semaphorin 3
(iii) an agent or a diagnosis kit for detecting alpha-synuclein and/or its aggregates, or
(iv) an agent or a diagnosis kit for genetically detecting a mutation associated with the appearance of Parkinson's disease and/or an allele associated with the more frequent appearance of Parkinson's disease.

## Revendications

1. Utilisation d'un composé selon la formule générale 1 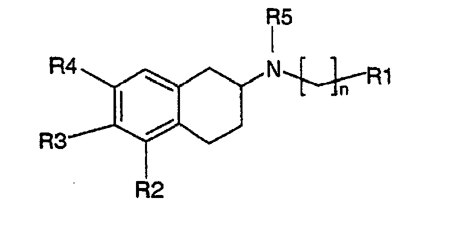 dans laquelle :
n=1-5;
R2 est = OA ; R3 et R4 sont choisis indépendamment parmi H et OA ;
où A est choisi parmi H, alkyle, alkoxyméthyle ou un groupe 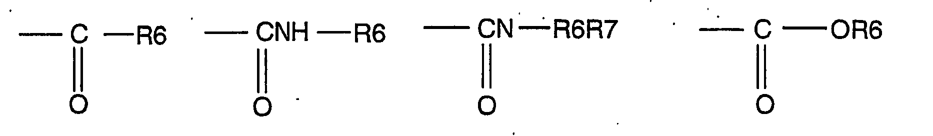 dans lequel R6 et R7 sont indépendamment un alkyle ou un aryle ;
R5 est un alkyle en C₁-C₃ ;
R1 est un groupe choisi parmi l'hydrogène, le 3-pyridyle, le 4-pyridyle, un phényle facultativement substitué, 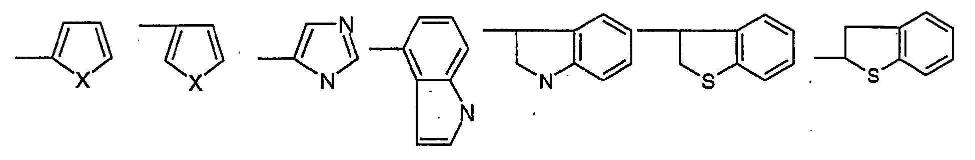
où X est choisi parmi S, O ou NH ;
le composé selon la formule I se présentant sous forme de racémate ou d'énantiomère (R) ou (S) pur ;
ainsi que des sels physiologiquement acceptables de ces composés, pour la fabrication d'un médicament destiné au traitement préventif de la maladie de Parkinson.

2. Utilisation selon la revendication 1, dans laquelle le traitement préventif est réalisé chez des sujets choisis parmi :
(a) des sujets sans symptômes parkinsoniens mais à risque accru de maladie de Parkinson, ou
(b) des sujets présentant des symptômes parkinsoniens précoces mais chez lesquels au moins trois des quatre symptômes cardinaux de la maladie de Parkinson (rigidité, tremblement au repos, bradykinésie, instabilité posturale) ne sont pas encore présents, ou seulement partiellement.

3. Utilisation selon la revendication 2, dans laquelle plusieurs des symptômes cliniques suivants peuvent être relevés chez les sujets selon (b) : troubles de l'odorat, dépression, troubles du sommeil du type "comportement onirique", constipation, anomalies motrices de courte durée.

4. Utilisation selon la revendication 2, dans laquelle les sujets présentent une mutation d'un gène PARK et/ou des modifications du schéma de la synucléine alpha ou de la neuromélanine.

5. Utilisation selon l'une quelconque des revendications précédentes, dans laquelle R3 et R4 représentent chacun l'hydrogène.

6. Utilisation selon l'une quelconque des revendications précédentes, dans laquelle A est un atome d'hydrogène ou un groupe choisi parmi 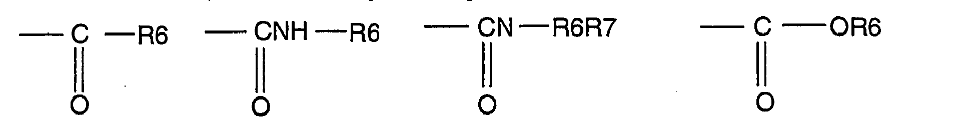 où R6 est un alkyle en C₁-C₁₂, un phényle ou un méthoxyphényle.

7. Utilisation selon l'une quelconque des revendications précédentes, dans laquelle n = 1-3.

8. Utilisation selon l'une quelconque des revendications précédentes, dans laquelle R1 est choisi parmi 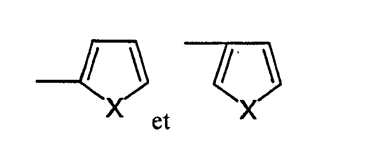 où X représente S, O ou NH.

9. Utilisation selon l'une quelconque des revendications précédentes, dans laquelle X est un atome de soufre.

10. Utilisation selon l'une quelconque des revendications précédentes, dans laquelle R5 représente un alkyle en C₃.

11. Utilisation selon l'une quelconque des revendications précédentes, dans laquelle R1 est un 2-thiényle, R3 et R4 sont tous les deux de l'hydrogène, R5 est un alkyle en C₃ et n=2.

12. Utilisation selon l'une quelconque des revendications précédentes, dans laquelle le composé est du 5,6,7,8-tétrahydro-6-[propyl[2-(2-thiényl)éthyl]amino]-1-naphtol.

13. Utilisation selon la revendication 12, dans laquelle le composé est l'énantiomère S pur (rotigotine).

14. Utilisation selon l'une quelconque des revendications précédentes, dans laquelle les sujets présentent, avant le début de l'administration du médicament, une perte de cellules dopaminergiques dans la substance noire inférieure à 60 %.

15. Utilisation selon l'une quelconque des revendications précédentes, dans laquelle les sujets présentent, avant le début de l'administration du médicament, un score UPDRS inférieur à 10.

16. Utilisation selon l'une quelconque des revendications précédentes, dans laquelle les sujets présentent un stade de Hoehn et Yahr de 0 ou 1.

17. Utilisation selon l'une quelconque des revendications précédentes, dans laquelle le médicament est destiné à une administration parentérale, transdermique ou muqueuse.

18. Utilisation selon l'une quelconque des revendications précédentes, dans laquelle le composé selon la formule générale I est administré à une dose de 0,05 à 50 mg par jour.

19. Kit pour le diagnostic et le traitement de la maladie de Parkinson, comprenant :
(a) un matériel de diagnostic permettant le diagnostic de la maladie de Parkinson ou d'un terrain exposant à la maladie de Parkinson à un stade précoce ou asymptomatique, et
(b) une formulation pharmaceutique comprenant des 2-aminotétralines substituées selon la formule générale I, telles qu'elles sont définies dans l'une des revendications 1 à 13.

20. Kit selon la revendication 19, dans lequel le matériel de diagnostic (a) est choisi parmi :
(i) un moyen ou un kit de diagnostic pour la mise en évidence de la neuromélanine,
(ii) un moyen ou un kit de diagnostic pour la mise en évidence de la sémaphorine 3,
(iii) un moyen ou un kit de diagnostic pour la mise en évidence de la synucléine alpha et/ou de ses agrégats, ou
(iv) un moyen ou un kit de diagnostic pour la mise en évidence génétique d'une mutation associée à la survenue de la maladie de Parkinson et/ou d'un allèle associé à une fréquence accrue de survenue de la maladie de Parkinson.
